(19) Europäisches Patentamt
European Patent Office
Office européen des brevets

(11) Veröffentlichungsnummer: **0 267 179 B1**

(12) **EUROPÄISCHE PATENTSCHRIFT**

(45) Veröffentlichungstag der Patentschrift: **17.02.93**

(51) Int. Cl.$^5$: **C07K 5/06**, A23L 1/305

(21) Anmeldenummer: **87890244.4**

(22) Anmeldetag: **04.11.87**

(54) **Neue Cystinverbindungen, ihre Herstellung und Verwendung.**

(30) Priorität: **07.11.86 AT 2980/86**

(43) Veröffentlichungstag der Anmeldung:
**11.05.88 Patentblatt 88/19**

(45) Bekanntmachung des Hinweises auf die Patenterteilung:
**17.02.93 Patentblatt 93/07**

(84) Benannte Vertragsstaaten:
**AT BE CH DE ES FR GB GR IT LI NL SE**

(56) Entgegenhaltungen:
**EP-A- 0 264 953**
**DE-A- 2 231 775**
**US-A- 3 794 633**

**THE CHEMISTRY OF POLYPEPTIDES, 1973, Seiten 63-85, Plenum Press; I. PHOTAKI: "On cysteine and cystine peptides"**

(73) Patentinhaber: **BBU-CHEMIE GESELLSCHAFT M.B.H.**

**A-9601 Arnoldstein(AT)**

Patentinhaber: **PFRIMMER KABI GMBH & CO. KG**
**Hofmannstrasse 26**
**W-8520 Erlangen(DE)**

(72) Erfinder: **Naderer,Dr. Rainer**
**Gailitz 18**
**A 9601 Arnoldstein(AT)**
Erfinder: **Langer, Dr. Klaus**
**Sudetenlandstr. 20**
**W 8520 Erlangen(DE)**
Erfinder: **Buxbaum, Dr. Lothar**
**Emailwerkstr. 14**
**A-9523 Landskron(AT)**

(74) Vertreter: **Collin, Hans, Dipl.-Ing. Dr. et al**
**Patentanwälte Dipl.-Ing. Dr. Hans Collin**
**Dipl.-Ing. Erwin Buresch Dipl.-Ing. Dr. Helmut Wildhack Dipl.-Ing. Armin Häupl Mariahilfer Strasse 50**
**A-1070 Wien (AT)**

**EP 0 267 179 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Cystinverbindungen, ihre Herstellung und Verwendung, vor allem als Bestandteil von Nutrienszusammensetzungen.

Für die Aufrechterhaltung der Lebensfunktionen von Menschen und Säugetieren muß dem Organismus unter anderem eiweißhältige Nahrung zugeführt werden, die vom Verdauungssystem in Aminosäuren umgewandelt wird. Die entstehenden Aminosäuren werden vom Körper zum Wachstum, für die Entwicklung, die Zellvermehrung und für Stoffwechselfunktionen benutzt.

Bei Ausfall oder bei Versagen des Verdauungssystems oder bei Patienten, die auf Grund verschiedener Einwirkungen stark geschwächt sind, wie z.B. nach Unfallverletzungen oder Operationen, aber auch im Falle spezieller Krankheitsbilder wie chronischem Nierenversagen oder Leberinsuffizienz, welche weit über das normale Maß hinausgehende Gaben an Aminosäuren erfordern, müssen freie Aminosäuren mittels entsprechender Präparate oral zugeführt werden.

Vielfach ist jedoch die Ernährung auf oralem Wege nicht oder nur unter erschwerten Umständen möglich. Diese Fälle treten besonders nach Operationen auf und in schweren Fällen von Krebs, Verbrennungen, Infektionen, Nierenversagen, Leberinsuffizienz sowie lang anhaltender Bewußtlosigkeit oder schweren Stoffwechselstörungen. In solchen Fällen müssen die Patienten dann parenteral mit Infusionslösungen ernährt werden, die neben Kohlenhydraten und Fett insbesondere auch Aminosäuren im geeigneten Verhältnis enthalten. Neben den als essentiell betrachteten Aminosäuren wie Lysin, Leucin, Isoleucin, Tryptophan, Methionin, Valin, Phenylalanin und Threonin ist bei bestimmten Personengruppen auch die Zufuhr anderer Aminosäuren unbedingt erforderlich.

Für Neugeborene und Säuglinge, sowie für Patienten mit bestimmten Erkrankungen (Leberzirrhose) ist z.B. die Aminosäure Cystein, bzw. deren oxidierte Form (Cystin) eine essentielle Aminosäure - d.h. sie kann nicht im Organismus aus der Aminosäure Methionin hergestellt werden, sondern muß als solche dem Organismus unmittelbar zugeführt werden. Die Aminosäure L-Cystein weist eine gute Wasserlöslichkeit (ca. 16 g/100 g Wasser) auf, ist aber gegen Hitzebehandlung nicht beständig, so daß sich cysteinhaltige Lösungen nicht ohne Zersetzung des Cysteins sterilisieren lassen. Die Aminosäure L,L-Cystin (welche vom Körper genauso verwertet werden kann wie L-Cystein) ist stabil gegenüber einer Hitzebehandlung, weist aber eine sehr geringe Wasserlöslichkeit (0,0095 g/100 g Wasser) auf, so daß ein Einsatz in einer Lösung für parenterale Ernährung nicht in Frage kommt.

Versuche zur Erhöhung der Wasserlöslichkeit des Cystins führten zu verschiedenen Di- und Tripeptiden, welche allerdings wegen ihrer geringen thermischen Stabilität für die übliche Herstellung von Infusionslösungen nicht geeignet sind. Einige kommen auch wegen ihres komplizierten Aufbaues und ihrer schwierigen Reinigung für den praktischen Einsatz kaum in Frage.

Auf der Suche nach als Bestandteil von Nutrienszusammensetzungen geeigneten Cystinderivaten wurde eine Gruppe von neuen diesbezüglichen Verbindungen der allgemeinen Formel

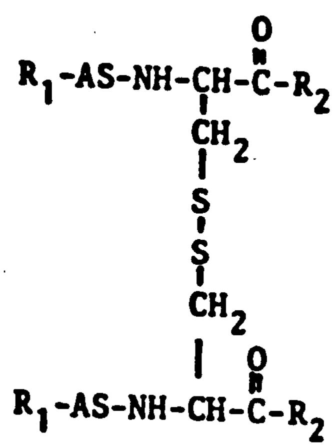

, (I)

gefunden, in der die AS unabhängig voneinander jeweils für eine natürlich vorkommende L-Aminosäure, insbesondere für Glycin, Alanin, Prolin, Threonin, Serin, Valin, Arginin, Lysin oder Ornithin, stehen; die $R_1$ unabhängig voneinander jeweils für Acetyl-, Propionyl-, Succinyl- oder Hydroxysuccinylstehen oder eine natürlich vorkommende L-Aminosäure bedeuten, die über ihre Carboxylgruppe amidartig mit der Aminogruppe der Aminosäure AS verknüpft ist, oder die beiden $R_1$ zusammen einen Bernsteinsäure- oder

Apfelsäurerest bilden; und die $R_2$ unabhängig voneinander jeweils für Methoxy oder Äthoxy, für den Hydroxyrest, wobei die im Falle der Bedeutung von $R_2$ = OH vorhandene(n) Carboxylgruppe(n) jeweils als solche oder als anorganisches oder organisches Salz, mit NaOH oder KOH, organischen Basen oder basischen Aminosäuren, wie L-Lysin, L-Ornithin, L-Arginin oder L-Histidin vorliegen kann (können), bzw. für den Rest einer über eine Amidgruppe gebundenen weiteren Aminosäure stehen, deren Carboxylgruppe(n) jeweils gegebenenfalls als anorganisches oder organisches Salz vorliegen kann (können).

Überraschenderweise hat sich herausgestellt, daß Verbindungen aus dieser Gruppe, und insbesondere das Bis-(acetylglycyl)-L,L-cystin und dessen Salze, sowohl gut wasserlöslich als auch stabil genug sind, um wärmesterilisierbar zu sein (z.B. Erhitzen auf 120°C während 5 min).

Die neuen Verbindungen der Formel I stellen somit u.a. einerseits wertvolle Bestandteile einer Nutrienszusammensetzung dar und anderseits ermöglichen sie deren einfache Sterilisierung.

Cystinpeptide ähnlicher Struktur sind im Aufsatz I.Photaki, on Cysteine and Cystine Peptides, in The Chemistry of Polypeptides, P.G. Katsoyannis ed. Plenum Press (1973) erwähnt. Dort ist auf symmetrische, unsymmetrische und zyklische Zystinpeptide und quervernetzte Dizystinpeptide hingewiesen.

Zur Herstellung der neuen Verbindungen kommen grundsätzlich die Peptidsynthesen und die Acylierung in Frage; so kann z.B. einerseits L,L-Cystin, ein L,L-Cystinalkylester oder ein L,L-Cystinpeptid, in dem an mindestens einer Carboxylgruppe des L,L-Cystins eine Amidbrücke zu einer weiteren Aminosäure ausgebildet ist, einer Peptidkupplung mit mindestens einer $\alpha$-aminoacylierten Aminosäure über die $\alpha$-Aminogruppen des L,L-Cystinderivats unterworfen werden.

Anderseits kann auch das mit einer $\alpha$-amino-unsubstituierten Aminosäure gebildete Cystinpeptid erst anschließend $\alpha$-aminoacyliert werden.

Für die Peptidkupplungsverfahren werden folgende Beispiele gegeben:

a) Aktivesterverfahren

Die $\alpha$-aminoacylierte Aminosäure wird mit 4-Nitrophenol in Gegenwart von Dicylohexylcarbodiimid als wasserabspaltendem Reagens in einem geeignetem Lösungsmittel (Acetonitril, Tetrahydrofuran oder Dimethylformamid) umgesetzt. Nach einer Reaktionszeit von 2 bis 96 Stunden wird der gebildete Harnstoff abfiltriert und nach dem Abdampfen des Lösungsmittels der 4-Nitrophenylester isoliert.

Der erhaltene Aktivester wird mit L,L-Cystin in Gegenwart von Natronlauge in einem geeignetem Lösungsmittel (wie z.B. Dimethylformamid, Acetonitril, Tetrahydrofuran) zum Peptid innerhalb von 2 bis 96 Stunden gekuppelt. Nach dem Ansäuern mit einer geeigneten Säure (Salzsäure oder Schwefelsäure) wird das Lösungsmittel abgedampft. Der Rückstand wird zwischen zwei miteinander nicht mischbaren Solventien (z.B. Wasser und Essigsäureethylester) verteilt, um das entstandene 4-Nitrophenol zu entfernen. Nach Abdampfen des Wassers wird aus der wässrigen Phase das gewünschte Endprodukt isoliert, welches dann durch Umkristallisation in einem geeigneten Solvens (z.B. Dimethylformamid Wasser, Ethanol) gereinigt wird.

b) Azlactonverfahren

Die $\alpha$-aminoacylierte Aminosäure wird in einem geeigneten Lösungsmittel (z.B. Dimethylformamid) mit einem wasserabspaltenden Reagens (z.B. Dicyclohexylcarbodiimid, Essigsäureanhydrid) zum 2-Alkyl-1,3-oxazolin-5-on kondensiert. Im Falle von Essigsäureanhydrid kann auch auf ein Lösungsmittel verzichtet werden, sodaß das Säureanhydrid selbst als Lösungsmittel wirkt. Nach der Reaktionszeit von 1 bis 96 Stunden wird der gegebenenfalls gebildete Harnstoff abfiltriert und anschließend das Lösungsmittel durch Abdampfen entfernt. Der Rückstand wird durch Umkristallisation in einem geeigneten Lösungsmittel (z.B. Ethanol) gereinigt. Das erhaltene Azlacton wird mit dem Natriumsalz von L,L-Cystin in einem geeigneten Lösungsmittel (z.B. Tetrahydrofuran, Dimethylformamid, Acetonitril) umgesetzt. Nach einer Reaktionszeit von 1 bis 96 Stunden wird das Peptid durch Ansäuern mit einer geeigneten Säure (z.B. Salzsäure oder Schwefelsäure) in Freiheit gesetzt. Nach Abdampfen des Lösungsmittels wird das Peptid durch Extrahieren mit einem geeigneten org. Solvens (z.B. Ethanol) von den anorganischen Bestandteilen abgetrennt. Nach Abdampfen des Solvens wird das Peptid durch Umkristallisation in einem geeigneten Lösungsmittel gereinigt.

c) Die $\alpha$-aminoacylierte Aminosäure kann direkt durch Kondensation mit L,L-Cystin-diester in Gegenwart eines wasserabspaltenden Reagens (z.B. Dicyclohexylcarbodiimid) in das entsprechende Peptid umgewandelt werden.

d) Die $\alpha$-aminoacylierte Aminosäure kann in das Säurechlorid, -anhydrid oder -azid umgewandelt werden, welche dann mit L,L-Cystin, bzw. dem entsprechenden Ester zum Peptid umgesetzt werden.

Die Acylierung eines bereits gebildeten L,L-Cystinpeptids an den $\alpha$-Aminopositionen wird am Beispiel der Herstellung von Bis-(acetylglycyl)-L,L-cystin aus dem Tripeptid Bis-glycyl-L,L-cystin erläutert: Das Tripeptid wird in einer äquimolaren Menge wässeriger Base gelöst und unter Kühlung wird eine entsprechende molare Menge Acetanhydrid zugesetzt, wobei noch eine entsprechende Menge an Base zugegeben

wird, um die bei der Reaktion freiwerdende Menge Essigsäure abzufangen. Nach Ablauf der Reaktion wird das Peptid durch Zugabe einer entsprechenden Menge Säure (z.B. Salzsäure oder Schwefelsäure) in Freiheit gesetzt. Nach Abdampfen des Wassers wird das acylierte Peptid durch Extraktion mit einem geeigneten Solvens (z.B. Ethanol, Aceton) abgetrennt. Das nach dem Abdampfen des Lösungsmittels gewonnene rohe Peptid wird durch Umkristallisation aus einem geeigneten Solvens (z.B. Ethanol, Aceton, Dimethylformamid, Acetonitril, Wasser) bzw. Kombinationen derselben gereinigt.

Es kommen alle üblichen Acylierungsmittel, u.a. auch Substanzen wie Essigsäure-4-nitrophenylester oder Thioessigsäure in Frage.

Demgemäß ist das Verfahren zur Herstellung der neuen Verbindungen der Formel I dadurch gekennzeichnet, daß in einem Lösungsmittel sowie in Gegenwart oder Abwesenheit einer Base, L,L-Cystin oder ein, insbesondere carboxylsubstituiertes, Cystinderivat

a) mit einem aktivierten Ester einer acylierten Aminosäure,

b) mit einem in 2-Stellung durch ein Alkyl, Aryl, Alkaryl oder Aralkyl substituierten 1,3-Oxazolin-5-onderivat (Azlacton),

c) mit einer acylierten Aminosäure in Gegenwart eines wasserabspaltenden Reagens, z.B. in Gegenwart von Dicyclohexylcarbodiimid oder von Äthoxyacetylen, oder

d) mit einem Säurechlorid, Säureanhydrid oder Azid einer acylierten Aminosäure umgesetzt wird, bzw. ein N,N'-bis-Aminosäure-L,L-cystin-derivat an mindestens einer $\alpha$-Aminogruppe acyliert wird und daß die so erhaltenen Verbindungen der Formel I gegebenenfalls mittels mindestens eines Bestandteils der Gruppe enthaltend anorganische und organische Basen sowie basische Aminosäuren in Salze umgewandelt werden.

Wenn die neuen Verbindungen der Formel I als Bestandteile von Nutrienszusammensetzungen Verwendung finden, werden sie in auf dem Pharmakasektor, z.B. zur Herstellung von festen und flüssigen Präparaten, oder Präparaten mit flüssigem Inhalt wie Kapseln, üblicher Weise formuliert.

So werden z.B. zur Herstellung von Infusionslösungen das gereinigte Peptid und die gewünschten Aminosäuren in destilliertem Wasser aufgelöst. Diesen Lösungen können auch noch Mineralsalze und andere Stoffe zugesetzt werden. Vor ihrer Verabreichung müssen sie isotonisch eingestellt werden. Bischer war es nicht möglich, derartige Lösungen ohne Zersetzung der cystinhaltigen Komponente in der üblichen Weise (5 min, 120 °C, pH = 5,5-7,5) zu sterilisieren. Durch die erfindungsgemäßen neuen Peptide ist eine Hitzebehandlung der vorstehend genannten Infusionslösung möglich, ohne daß es zu einer Zersetzung des Cystinderivats kommt.

Werden diese Infusionslösungen gemeinsam mit Kohlehydraten (z.B. Glucose) angewandt, so müssen Kohlehydrat- und Aminosäurenlösung getrennt sterilisiert werden, da es sonst zu einer Maillardreaktion (und nachfolgend zu einer Verfärbung) der Lösung kommt.

Die beschriebenen Cystinderivate eignen sich auch für die Herstellung von oralen Aminosäurepräparaten, welche z.B. als Tabletten, Granulate, Dragees oder Kapseln, z.B. mit Ölphase gefüllte Kapseln, eingenommen werden können.

Nachfolgend werden einige Beispiele für die Herstellung und Prüfung der erfindungsgemäßen Cystinpeptidderivate gegeben:

## Beispiel 1:

Synthese von Bis-(benzyloxycarbonylglycyl)-L,L-cystin

35,3 g L,L-Cystin (0,147 Mol) werden in 340 ml 1N Natronlauge gelöst, auf unter +10 °C abgekühlt und eine Lösung von 90 g n-Benzyloxycarbonylglycin-hydroxysuccinimid-ester (0,294 Mol) in 350 ml Aceton zugetropft. Nach Gesamtreaktionszeit von 12 h wird das Aceton durch Abdampfen im Vakuum entfernt. Durch Ansäuern mit verdünnter Schwefelsäure wird das Produkt in Freiheit gesetzt; es wird mit einem organischen Lösungsmittel extrahiert und aus einem geeigneten Lösungsmittelgemisch umkristallisiert.

| | |
|---|---|
| Ausbeute: | 60 g (65 % d.Th.) |
| Fp: | 135 - 140 °C |
| DC (Dünnschichtchromatographie) | (n-Butanol/Eisessig/Wasser 60/30/30): Rf = 0,6 |
| | (n-Propanol/Ammoniak 2/1): Rf = 0,7 |

Beispiel 2:

Synthese von Bis-glycyl-L,L-cystin

50 g Bis-(Benzyloxycarbonylgycyl)-L,L-cystin (0,08 Mol) werden mit 125 ml 33 %iger Bromwasserstoffsäure in Eisessig übergossen. Nach einstündigem Rühren wird mit 1 l Diethyläther versetzt und in der Kälte zur Kristallisation abgestellt. Das ausgefallene kristalline Produkt wird durch Filtration gesammelt und durch Waschen mit Diethyläther benzylbromidfrei gewaschen. Das getrocknete Produkt wird in Wasser aufgelöst und durch Zugabe einer Base wird das Peptid aus dem Hydrobromid in Freiheit gesetzt. Nach Entfernen des Lösungsmittels, wird das Produkt durch Behandeln mit einem organischen Lösungsmittel (z.B. Dimethylformamid, Acetonitril, Methanol) gereinigt.

| | |
|---|---|
| Ausbeute: | 22 g (70 % d.Th.) |
| $[\alpha]_{20}^{D}$ : | - 116° (C = 1 in Wasser) |
| DC | (n-Butanol/Eisessig/Wasser 60/30/30):Rf = 0,1 |
| | (n-Propanol/Ammoniak 2/1): Rf = 0,05 |

Beispiel 3:

Synthese von Bis-(acetyl-glycyl)-L,L-cystin:

3,5 g Bis-glycyl-L,L-cystin (0,01 Mol) werden in 20 ml 1N Natronlauge gelöst und auf unter +10°C abgekühlt; gleichzeitig werden 3,1 g Acetanhydrid (0,03 Mol) und 55 ml 1N Natronlauge zugesetzt. Nach einer Gesamtreaktionszeit von 2 h wird durch Säurezugabe auf pH 1-2 gestellt. Nach Abdampfen des Wassers im Vakuum wird das Produkt durch Extraktion mit einem geeigneten organischen Lösungsmittel (z.B. einem Alkohol) vom anorganischen Salz abgetrennt. Nach Entfernen des Lösungsmittels im Vakuum wird der Rückstand aus Ethanol/Aceton umkristallisiert.

| | |
|---|---|
| Ausbeute: | 3,1 g (70 % d.Th.) |
| Fp: | 124 - 134°C |
| $[\alpha]_{20}^{D}$ : | - 124,5° (C = 1 im Wasser) |
| DC | (Ethanol/Wasser 9/1): Rf = 0,67 |

Beispiel 4:

Überprüfung der Wasserlöslichkeit und Hitzestabilität von Bis-(acetylglycyl)-L,L-cystin

0,5 g Bis-(acetylglycyl)-L,L-cystin werden in 50 ml destilliertem Wasser gelöst. Durch Zugabe einer 1N Natriumhydrogencarbonatlösung wird auf pH 6,5 gestellt und anschließend mit destilliertem Wasser auf 100 ml aufgefüllt. Von dieser Lösung wird sofort ein HPLC-Chromatogramm aufgenommen. Anschließend wird die Lösung im siedenden Wasserbad erhitzt. Nach 10, 20 und 30 min Erhitzungsdauer zeigen sich keine wesentlichen Veränderungen im HPLC-Chromatogramm.

Beispiel 5:

Die in der folgenden Tabelle angeführten Ingredienzien wurden in destilliertem Wasser gelöst und in Infusionsflaschen abgefüllt. Diese wurden anschließend sterilisiert.

| Peptid und Aminosäuren | g/1000 ml |
|---|---|
| Bis-(acetylglycyl)-L,L-cystin | 1,4 |
| L-Isoleucin | 2,5 |
| L-Leucin | 2,8 |
| L-Lysin | 1,5 |
| L-Methionin | 0,8 |
| L-Phenylalanin | 1,4 |
| L-Threonin | 1,7 |
| L-Tryptophan | 0,56 |
| L-Valin | 2,1 |
| L-Arginin | 3,5 |
| L-Histidin | 0,7 |
| L-Alanin | 5,5 |
| L-Asparagin | 4,0 |
| Glycin | 0,5 |
| L-Prolin | 3,8 |
| L-Ala-L-Tyr 1) | 1,5 |

1) Dipeptid aus L-Alanin und L-Tyrosin

Beispiel 6:

Herstellung von n-Acetyl-glycin-4-nitrophenylester

29,3 g N-acetylglycin (0,25 Mol) und 34,8 g 4-Nitrophenol (0,25 Mol) werden in 250 ml Dimethylformamid (DMF) gelöst. Es wird unter +10°C abgekühlt und 51,6 g Dicyclohexylcarbodiimid (0,25 Mol) werden zugegeben. Nach einer Reaktionszeit von 12 h wird vom ausgefallenen Dicyclohexylharnstoff abfiltriert und das DMF im Vakuum abgedampft. Der Rückstand wird aus einem geeigneten Lösungsmittelgemisch (z.B. Methanol/Wasser oder Ethylacetat/Diethylether) umkristallisiert.

Ausbeute: 38 g (64 % d.Th.)
Fp: 120 - 123°C
DC (Ethanol/Wasser): Rf = 0,8

Beispiel 7:

Herstellung von Bis-(acetyl-glycyl)-L,L-cystindimethylester

9,5 g n-Acetyl-glycin-4-nitrophenylester (0,04 Mol) und 6,8 g L,L-Cystindimethylester-dihydrochlorid (0,02 Mol) werden in 100 ml DMF suspendiert. Nach Abkühlen auf unter +10°C werden 4,1 g Triethylamin (0,04 Mol) zugesetzt. Nach 48 h Reaktionszeit wird das Dimethylformamid im Vakuum abgedampft. Aus dem Rückstand wird das Produkt durch Extraktion mit geeignetem organischen Lösungsmittel gewonnen.

Ausbeute: 5,6 g (50 % d.Th.)

Beispiel 8:

Herstellung von Bis-(acetyl-glycyl)-L,L-cystin über 2-Methyl-1,3-oxazolon-5 (Azlacton)

4,8 g L,L-Cystin (0,02 Mol) werden in 1N Natronlauge gelöst. 4 g 2-Methyl-1,3-oxazolon-5 (0,04 Mol) in einem inerten Lösungsmittel läßt man zutropfen. Nach 12 h Reaktionszeit wird angesäuert und das Lösungsmittel im Vakuum entfernt. Der Rückstand wird umkristallisiert.

Ausbeute: 5.7 g (65 % d.Th.)
Physikalische Daten: Siehe Beispiel 3

Beispiel 9:

Herstellung von Bis-(acetylglycyl)-L,L-cystin-dimethylester über Carbodiimid

4,7 g Acetylglycin (0,04 Mol) werden mit 6,8 g L,L-Cystin-dimethylesterdihydrochlorid (0,02 Mol) in Gegenwart von 8,3 g Dicyclohexylcarbodiimid (0,04 Mol) in DMF als Lösungsmittel umgesetzt.
Nach Abfiltrieren des Harnstoffs wird der Rückstand umkristallisiert.

Ausbeute: 5,3 g (60 % d.Th.)

Beispiel 10:

Herstellung von Bis-(acetyl-glycyl)-L,L-cystin:

36 g L,L-Cystin (0,15 Mol) werden mit 60,7 g Triethylamin (0,6 mol) in 300 ml Wasser gelöst.
64,3 g N-Acetyl-glycin-hydroxysuccinimidester (0,3 Mol) in Dimethylformamid werden unter Kühlung zugetropft.
Nach dem Ansäuern wird das Lösungsmittel entfernt und der Rückstand umkristallisiert.

Ausbeute: 48,1 g (73 % d. Th.)
Fp: 124-134 ° C
$(\alpha)_{20}^{D}$ : - 124,5 ° (c = 1 in Wasser)
DC (Ethanol/Wasser 9/1): $R_f$ = 0,67

Beispiel 11:

Herstellung von Bis-(acetyl-glycyl)-bis-arginat:

Eine 10%-ige wässrige Lösung von Bis-acetylglycyl-L,L-cystin wird mit einer 10%-igen wässrigen Lösung von L-Arginin vermischt, bis das Gemisch neutral reagiert. Das Wasser wird abgedampft und der Rückstand durch Behandeln mit einen organischen Lösungsmittel gereinigt.

Fp: 155-165 ° C
$(\alpha)_{20}^{D}$ : - 74,5 ° (C = 1 in Wasser)

Beispiel 12:

Herstellung von Bis-acetyl-L-tyrosyl-L,L-cystin

2,27 g Bis-L-tyrosyl-L,L-cystin (0,004 Mol) werden in Wasser suspendiert und eine Lösung von 1,57 g Essigsäurehydroxysuccinimidester (0,010 Mol) in Dioxan zugetropft. Der pH-Wert wird durch Zugabe von wässriger Natronlauge zwischen pH 6 und pH 10 konstant gehalten. Nach dem Ansäuern wird das Lösungsmittel entfernt und das erhaltene Produkt aus Wasser umkristallisiert.

Fp.: 141-146 ° C
$(\alpha)_{20}^{D}$ : - 89,3 ° (c = 1 in Wasser)
DC (Ethanol/Wasser 8/2): $R_f$ = 0,67

Beispiel 13:

Herstellung von Bis-propionyl-glycyl-L,L-cystin.

7,8 g Bis-glycyl-L,L-cystin (0,02 Mol) werden in Wasser gelöst und mit 0,9 Mol einer organischen Base versetzt. Es werden unter Kühlung 5,21 g Propionsäureanhydrid (0,04 Mol) zugetropft.
Nach dem Ansäuern wird das Lösungsmittel entfernt und der Rückstand umkristallisiert.

Ausbeute: 8 g (86 % d. Theorie)
Fp: 133-145 ° C
$(\alpha)_{20}^{D}$ : -128,8 ° (c = 2 in 1N $NaHCO_3$)
DC (Ethanol/Wasser 8/2): $R_f$ = 0,63

Beispiel 14

Herstellung von Bis-acetyl-glycyl-L,L-cystin-dimethylester

3,4 g L,L-Cystindimethylesterdihydrochlorid (0,01 Mol) und 13,3 g N-Methylmorpholin (0,13 Mol) werden in Ethanol gelöst. Unter Kühlung wird mit 4,3 g Acetylglycinhydroxysuccinimidester (0,02 Mol) versetzt. Das Lösungsmittel wird entfernt und der Rückstand aus einem organischen Lösungsmittelgemisch umkristallisiert.

| | |
|---|---|
| Fp.: | 155-160 °C |
| $(\alpha)_{20}^{D}$ : | - 92,5 ° (c = 1 in Wasser) |
| DC | (n Butanol/Eisessig/Wasser 2/1/1): $R_f$: = 0,45 |

Beispiel 15:

Herstellung von Bis-acetyl-L-alanyl-L,L-cystin

6,9 g Bis-L-alanyl-L,L-cystin (0,018 Mol) werden in 300 ml Ethanol-Wasser-Mischung suspendiert. 6,1 g Essigsäurehydroxysuccinimidester (0,040 Mol) - gelöst in Dioxan - werden zugetropft. Der pH-Wert wird durch Zugabe von Natronlauge konstant gehalten. Nach dem Ansäuern wird das Lösungsmittel entfernt und das erhaltene Produkt durch Umkristallisation gereinigt.

| | |
|---|---|
| Ausbeute: | 6,2 g (84 % d. Th.) |
| Fp.: | 194-197 °C |
| $(\alpha)_{20}^{D}$ : | - 181 ° (c = 1 in Wasser) |
| DC | (Ethanol/Wasser): $R_f$: = 0,42 |

Beispiel 16:

Herstellung von Bis-glycyl-glycyl-L,L-cystin

11,7 g Bis-glycyl-L,L-cystin (0,03 Mol) werden mit 12,6 g Natriumhydrogencarbonat (0,15 Mol) in Wasser gelöst. Unter Kühlung werden 16,3 g N-tert. Butyloxycarbonyl-glycinhydroxysuccinimidester (0,06 Mol) in einem geeigneten Lösungsmittel zugesetzt. Nach einer Reaktionszeit zwischen 6 und 48 Stunden wird angesäuert und das Lösungsmittel entfernt. Der Rückstand wird aus einem Lösungsmittelgemisch umkristallisiert. Durch Zugabe von Trifluoressigsäure wird die tert. Butyloxycarbonylschutzgruppe abgespalten und durch Zugabe von Ammoniak wird das Peptid in Freiheit gesetzt.

| | |
|---|---|
| Ausbeute: | 7,3 g (52 % d. Th.) |
| Fp.: | 215-218 °C (Zersetzung) |
| $(\alpha)_{20}^{D}$ : | - 108,9 ° (c = 1 in 1N HCl) |

Beispiel 17:

Herstellung von Bis-N-succiniyl-glycyl-L,L-cystin

3,9 g Bis-glycyl-L,L-cystin (0,01 Mol) werden in Wasser gelöst und bei konstantem pH-Wert mit einer Lösung von 2,0 g Bernsteinsäureanhydrid (0,02 Mol) in einem geeigneten Lösungsmittel versetzt. Nach einer Reaktionszeit von einer Stunde wird angesäuert, das Lösungsmittel entfernt und der Rückstand umkristallisiert.

| | |
|---|---|
| Ausbeute: | 5 g (90 % d. Th.) |
| Fp.: | 105-115 °C |
| $(\alpha)_{20}^{D}$ : | - 88,5 ° (c = 1 in Wasser) |
| DC | (Chloroform/Methanol/Eisessig 5/3/1): $R_f$ = 0,50 |

Beispiel 18:

Herstellung von Bis-acetyl-glycyl-L,L-cystin-bis-L-tyrosinethylester-salz

L-Tyrosinethylesterhydrochlorid wird in Wasser aufgelöst und durch Zugabe von Natriumcarbonat wird der Ester in Freiheit gesetzt. Dieser wird in einem Lösungsmittel aufgenommen (z.B. Essigsäureethylester). 8,9

g Bis-acetyl-glycyl-L,L-cystin (0,02 Mol) werden in Wasser gelöst und mit einer äquivalenten Menge der organischen Lösung des Tyrosinethylesters versetzt. Nach mehrmaligem Waschen der wässrigen Phase mit einem organischen Solvens wird das Wasser abgedampft. Der Rückstand wird aus einem organischen Lösungsmittelgemisch umkristallisiert.

Ausbeute: 14,2 g (81 % d. Th.)
Fp.: 165-170 °C
$(\alpha)_{20}^{D}$ : - 82,2 ° (c = 1 in Wasser)

**Patentansprüche**

1. Neue Cystinverbindungen der allgemeinen Formel

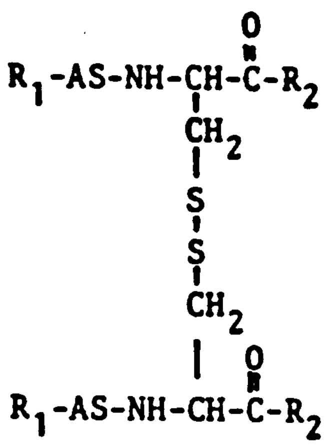

, (I)

in der die AS unabhängig voneinander jeweils für eine natürlich vorkommende L-Aminosäure, insbesondere für Glycin, Alanin, Prolin, Threonin, Serin, Valin, Arginin, Lysin oder Ornithin, stehen; die $R_1$ unabhängig voneinander jeweils für Acetyl-, Propionyl-, Succinyl- oder Hydroxysuccinyl- stehen oder eine natürlich vorkommende L-Aminosäure bedeuten, die über ihre Carboxylgruppe amidartig mit der Aminogruppe der Aminosäure AS verknüpft ist, oder die beiden $R_1$ zusammen den Bernsteinsäure- oder Apfelsäurerest bilden; und die $R_2$ unabhängig voneinander jeweils für Methoxy oder Äthoxy, für den Hydroxyrest, wobei die im Falle der Bedeutung von $R_2$ = OH vorhandene(n) Carboxylgruppe(n) jeweils als solche oder als anorganisches oder organisches Salz, mit NaOH oder KOH, organischen Basen oder basischen Aminosäuren, wie L-Lysin, L-Ornithin, L-Arginin oder L-Histidin vorliegen kann (können), bzw. für den Rest einer über eine Amidgruppe gebundenen weiteren Aminosäure stehen, deren Carboxylgruppe(n) jeweils gegebenenfalls als anorganisches oder organisches Salz vorliegen kann (können).

2. Verfahren zum Herstellen von neuen Cystinverbindungen nach Anspruch 1, insbesondere von Bis-(acetylglycyl)-L,L-cystin, dadurch gekennzeichnet, daß, in einem Lösungsmittel sowie in Gegenwart oder Abwesenheit einer Base, L,L-Cystin oder ein, insbesondere carboxylsubstituiertes, Cystinderivat
   a) mit einem aktivierten Ester einer acylierten Aminosäure,
   b) mit einem in 2-Stellung durch ein Alkyl, Aryl, Alkaryl oder Aralkyl substituierten 1,3-Oxazolin-5-onderivat (Azlacton),
   c) mit einer acylierten Aminosäure in Gegenwart eines wasserabspaltenden Reagens, z.B. in Gegenwart von Dicyclohexylcarbodiimid oder von Äthoxyacetylen, oder
   d) mit einem Säurechlorid, Säureanhydrid oder Azid einer acylierten Aminosäure umgesetzt wird,
   bzw. ein N,N′-bis-Aminosäure-L,L-cystin-derivat an mindestens einer $\alpha$-Aminogruppe acyliert wird und daß die so erhaltenen Verbindungen der Formel I gegebenenfalls mittels mindestens eines Bestandteils der Gruppe enthaltend anorganische und organische Basen sowie basische Aminosäuren in Salze umgewandelt werden.

3. Nutrienszusammensetzung, dadurch gekennzeichnet, daß sie mindestens eine Cystinverbindung nach Anspruch 1, vorzugsweise Bis-(acetylglycyl)-L,L-cystin oder dessen Salze, insbesondere in Massenanteilen von 0,1 bis 20, vorzugsweise 1 bis 10 %, bezogen auf die Zusammensetzung enthält und

insbesondere als wässerige Lösung vorliegt.

**4.** Verfahren zur Herstellung einer Nutrienszusammensetzung nach Anspruch 3, dadurch gekennzeichnet, daß man mindestens eine Cystinverbindung nach Anspruch 1, vorzugsweise Bis-(acetylglycyl)-L,L-cystin oder dessen Salze, gegebenenfalls unter Zusatz mindestens eines weiteren Nährstoffs wie z.B. Polyole, insbesondere Sorbit oder Xylit, Fette, Glucose und Oligosaccharide, sowie gegebenenfalls unter Zusatz von Mineralsalzen, Spurenelementen, Vitaminen und/oder in Nutrienszusammensetzungen weiterhin üblichen Bestandteilen in eine feste oder flüssige Verabreichungsform überführt, vorzugsweise eine wässerige Lösung bildet.

## Claims

**1.** A new cystine compound of the general formula

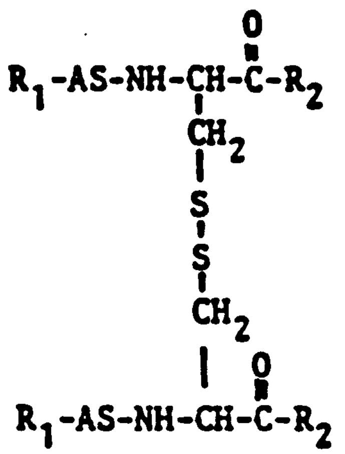

, (I)

wherein the AS independently of each other each stand for a naturally occurring L-amino acid, in particular for glycine, alanine, proline, threonine, serine, valine, arginine, lysine or ornithine; the $R_1$ independently of each other each stand for acetyl, propionyl, succinyl or hydroxy succinyl or indicate a naturally occuring L-amino acid which is linked to the amino group of the AS amino acid through its carboxyl group in an amide-like manner, or the two $R_1$ together form the succinic acid or malic acid residue; and the $R_2$ independently of each other each stand for methoxy or ethoxy, for the hydroxy residue, the carboxyl group(s), in the case of the meaning of $R_2$ = OH, existing as such or as an inorganic or organic salt, with NaOH or KOH, organic bases or basic amino acids, like L-lysine, L-ornithine, L-arginine or L-histidine and stand for the residue of an additional amino acid bound by an amide group, respectively, the carboxyl group(s) of which each may optionally be an inorganic or organic salt.

**2.** A method for the preparation of new cystine compounds according to claim 1, in particular of bis-(acetylglycyl)-L,L-cystine, characterized in that, in a solvent as well as in the presence or absence of a base, L,L-cystine or an in particular carboxyl-substituted cystine derivative is reacted with

a) an ac tivated ester of an acylated amino acid,

b) a 1,3-oxazoline-5-on-derivate (azlactone) substituted by an alkyl, aryl, alkaryl or aralkyl in position 2,

c) an acylated amino acid in the presence of a dehydrating reagent, e.g. in the presence of dicyclohexyl carbodiimide or of ethoxy acetylene, or

d) an acid chloride, acid anhydride or azide of an acylated amino acid,

and an N,N'-bis-amino acid-L,L-cystine-derivative is acylated at at least one $\alpha$-amino group, respectively, and in that the thus obtained compound according to formula I is transformed into salts optionally by means of at least one component of the group containing inorganic and organic bases as well as basic amino acids.

3. A nutrient composition, characterized in that it contains at least one cystine compound according to claim 1, preferably bis-(acetylglycyl)-L,L-cystine or its salts, in particular in percentages by mass of 0,1 to 20, preferably 1 to 10, on the basis of the composition, and in particular is in the form of an aqueous solution.

4. A method for the preparation of a nutrient composition according to claim 3, characterized in that at least one cystine compound according to claim 1, preferably bis-(acetylglycyl)-L,L-cystine or its salts, optionally adding at least one additional nutrient as e.g. polyols, in particular sorbitol or xylitol, fats, glucose and oligosaccharides, as well as optionally adding mineral salts, trace elements, vitamines and/or other conventional components of nutrient compositions, is converted into a solid or liquid formulation for administration, preferably being formed as an aqueous solution.

**Revendications**

1. Nouveaux composés de cystine de la formule générale

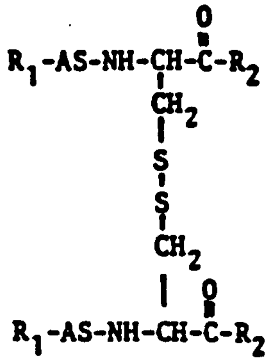

, (I)

dans laquelle chaqu'un des AS représente, indépendamment des autres, un L-aminoacide d'apparition naturelle, en particulier la glycine, l'alanine, la proline, la thréonine, la sérine, la valine, l'arginine, la lysine ou l'ornithine; chaqu'un des $R_1$ représente, indépendamment des autres, l'acétyle, le propionyle, le succinyle ou l'hydroxysuccinyle ou un L-aminoacide d'apparition naturelle, qui est lié avec l'amino-groupe de l'aminoacide AS par son groupe carboxyle de façon amidique; ou les deux $R_1$ ensemble forment le radical acide succinique ou acide malique; et chaqu'un des $R_2$ représente, indépendamment des autres, le méthoxyl ou l'éthoxyl, le radical hydroxy, le(s) groupe(s) carboxyle(s) pouvant etre présent(s) comme tel(s) ou comme sel inorganique ou organique avec NaOH ou KOH, bases organiques ou aminoacides basiques, par exemple L-lysine, L-ornithine, L-arginine ou L-histidine en cas de la signification $R_2$ = OH, ou bien représentent le radical d'un aminoacide de plus, lié par un groupe amide, dont le(s) groupe(s) carboxyle(s) peut/peuvent, le cas échéant, peut/peuvent etre présent(s) comme sel inorganique ou organique.

2. Procédé pour la production de nouveaux composés de cystine selon la revendication 1, en particulier de bis-(acétyle glycyle)-L,L-cystine, characterisé en ce que, dans un solvant et en présence ou en absence d'une base, l'L-L-cystine ou un dérivé de cystine, en particulier avec substitution carboxylique, est fait réagir

a) avec un ester activé d'un aminoacide acylé,

b) avec un derivé d'1,3-oxazoline-5-one (azlactone) substitué en position 2 par un alkyle, aryle, alkaryle ou aralkyle,

c) avec un aminoacide acylé en présence d'un réactif déshydrogénant, par exemple en présence de dicyclo-hexyle-carbodiimide ou d'éthoxy-acétylène, ou

d) avec un chlorure d'acide, anhydride d'acide ou acide d'un aminoacide acylé,

ou bien un derivé de N,N'-bis-aminoacide-L,L-cystine est acylé dans au moins un α-amino-groupe et en ce que les composés de la formule I ainsi obtenus sont transformés en sels, le cas échéant au moyen d'au moins un composant du group comprenant des bases inorganiques ou organiques ainsi que des aminoacides basiques.

**3.** Composition nutritive characterisée en ce qu'elle comporte au moins un composé de cystine selon la revendication 1, de préférence le bis-(acétyle-glycyle)-L,L-cystine ou ses sels, en particulier en pourcentages de masse de 0,1 à 20, de préférence 1 à 10 %, par rapport à la composition, et est présent en particulier comme solution aqueuse.

**4.** Procédé de production d'une composition nutritive selon la revendication 3, characterisé en ce que au moins un composé de cystine selon la revendication 1, de préférence le bis-(acétyle-glycyle)-L,L-cystine ou ses sels, le cas échéant avec addition d'au moins une substance nutritive de plus, par example des polyols, en particulier le sorbitol ou le xylitol, des lipides, de la glucose, des oligosaccharides, aussi bien que, le cas échéant, avec addition de sels minéraux, d'éléments de trace, de vitamines et/ou d'autres composants usuels dans les compositions nutritives est transformé en forme d'aministration solide ou liquide, de préférence formant une solution aqueuse.